# EUROPEAN PATENT APPLICATION

(11) **EP 4 305 961 A1**
(43) Date of publication of application: **17.01.2024**
(21) Application number: 22767276.3
(22) Date of filing: 11.03.2022
(51) Int. Cl.: A01N 41/04, A01N 25/10, A01P 3/00

(54) **VIRAL INFECTION INHIBITOR AND VIRAL INFECTION-INHIBITING PRODUCT**

(30) Priority: 12.03.2021 JP 2021040825
(71) Applicant: SEKISUI CHEMICAL CO., LTD., Osaka-shi Osaka 530-8565 (JP)
(72) Inventor: NISHIHARA, Kazuya, Mishima-gun, Osaka 618-0021 (JP); SUZUKI, Taro, Mishima-gun, Osaka 618-0021 (JP); KAWAMURA, Daichi, Mishima-gun, Osaka 618-0021 (JP)
(74) Representative: Ter Meer Steinmeister & Partner
(86) International application number: PCT/JP2022/010983
(87) International publication number: WO 2022/191322

(57) **Abstract**

The present invention provides a viral infection inhibitor capable of exhibiting an excellent viral infection inhibitory effect. The viral infection inhibitor of the present invention is characterized by including a compound having a salt of a sulfonic acid group and an organic acid, The organic acid preferably has a solubility of 20 g/L or less in water at 25°C, and therefore, the viral infection inhibitor has an excellent viral infection inhibitory effect against both enveloped viruses and non-enveloped viruses, and exhibits the viral infection inhibitory effect against various types of viruses.

## Description

### Technical Field

The present invention relates to a viral infection inhibitor and a viral infection-inhibiting product.

### Background Art

In recent years, in addition to a seasonal influenza virus epidemic, a novel coronavirus (COVID-19) has caused a global pandemic.

It has also been confirmed that a highly pathogenic avian influenza virus has mutated and is now transmissible through humans. Furthermore, there is a concern about a SARS virus, which has an extremely high fatality rate. Thus, general anxiety about viruses is constantly increasing.

To address these problems, Patent Literature 1 proposes an antiviral synthetic resin composition including 0.5 parts by weight or more of a sulfonic acid-based surfactant relative to 100 parts by weight of the synthetic resin.

### Citation List

### Patent Literature

PTL 1: Japanese Patent Application Laid-Open No. 2016-128395

### Summary of Invention

### Technical Problem

However, the above-mentioned antiviral synthetic resin composition merely includes, in the synthetic resin, a sulfonic acid-based surfactant that does not have sufficient antiviral properties (viral infection inhibitory effect). Thus, a viral infection inhibitor having an excellent viral infection inhibitory effect is desired.

The present invention provides a viral infection inhibitor capable of exhibiting an excellent viral infection inhibitory effect.

### Solution To Problem

A viral infection inhibitor of the present invention is characterized by including a compound having a salt of a sulfonic acid group and an organic acid.

A viral infection-inhibiting product of the present invention is characterized by including:
a base material; and
the viral infection inhibitor included in the base material.

### Advantageous Effects of Invention

The viral infection inhibitor of the present invention includes a compound having a salt of a sulfonic acid group and an organic acid, and thus exhibits an excellent viral infection inhibitory effect against both enveloped viruses and non-enveloped viruses, and also exhibits a viral infection inhibitory effect against various types of viruses. Description Of Embodiments

The viral infection inhibitor of the present invention includes a compound having a salt of a sulfonic acid group and an organic acid as active ingredients.

### [Compound having salt of sulfonic acid group]

The compound having a salt of a sulfonic acid group has a salt of a sulfonic acid group within the molecule. The compound having a salt of a sulfonic acid group exhibits a viral infection inhibitory effect, which is derived from a molecular structural moiety containing the salt of the sulfonic acid group (-SO₃X: X is a metallic element or NH₄⁺). The compound having a salt of a sulfonic acid group has an excellent viral infection inhibitory effect, in particular, against enveloped viruses. It should be noted that the compound having a salt of a sulfonic acid group may have a carboxy group (-COOH), a thiol group (-SH), and a hydroxy group (-OH).

The salt of the sulfonic acid group is not particularly limited, and examples thereof include a sodium salt, a calcium salt, an ammonium salt, a magnesium salt of a sulfonic acid, and a barium salt of a sulfonic acid. Among these, a sodium salt is preferable.

The compound having a salt of a sulfonic acid group is preferably an organic compound. In the present invention, an organic compound refers to a compound containing at least one (preferably 2 or more) carbon atom within a molecule and containing a carbon-hydrogen bond (C-H bond).

When the compound having a salt of a sulfonic acid group is an organic compound, affinity with the organic acid, which will be described later, is enhanced. As a result, the organic acid and the compound having a salt of a sulfonic acid group become in close proximity to each other, and the interaction between the organic acid and the compound having a salt of a sulfonic acid group is enhanced, which may lead to an enhancement in the viral infection inhibitory effect against both a virus having no envelope (non-enveloped virus) and a virus having an envelope (enveloped virus).

Furthermore, when the viral infection inhibitor is caused to adhere to the surface of the resin particles, if an organic compound is used as the compound having a salt of a sulfonic acid group, the compound having a salt of a sulfonic acid group has hydrophobicity in an organic chain moiety (hydrophobic moiety) represented by a carbon-hydrogen bond moiety. In the compound having a salt of a sulfonic acid group, the organic chain moiety has excellent affinity with the resin particles. Meanwhile the salt of the sulfonic acid group, which has lower affinity with the resin particles than does the organic chain moiety, tends to face outward, which may allow the viral infection inhibitor to more effectively exhibit the viral infection inhibitory effect.

The compound having a salt of a sulfonic acid group preferably has an aromatic ring. In the compound having a salt of a sulfonic acid group, having an aromatic ring enhances affinity with the organic acid. As a result, the organic acid and the compound having a salt of a sulfonic acid group become in close proximity to each other, and the interaction between the organic acid and the compound having a salt of a sulfonic acid group is enhanced, which may lead to an enhancement in the viral infection inhibitory effect against both enveloped viruses and non-enveloped viruses.

The viral infection inhibitor or the compound having a salt of a sulfonic acid group constituting the viral infection inhibitor may be caused to adhere to the surface of the resin particles and used. In this case, when the compound having a salt of a sulfonic acid group has an aromatic ring, the compound having a salt of a sulfonic acid group maintains a state of strongly adhering to the surface of the resin particle, due to the affinity between the aromatic ring and the synthetic resin constituting the resin particles, and the compound is prevented from detaching from the resin particle. This effect allows the viral infection inhibitor to maintain an excellent viral infection inhibitory effect over a long period of time.

The aromatic ring may be a monocyclic aromatic ring or may be obtained by condensing monocyclic aromatic rings (condensed aromatic ring). The aromatic ring is not particularly limited, and examples thereof include a benzene ring, a naphthalene ring, an anthracene ring, a biphenyl, and a phenoxyphenyl. Among these, a benzene ring and a naphthalene ring are preferable. In an aromatic ring, any one or more hydrogen atoms of an aromatic ring and a condensed aromatic ring are abstracted and covalently bonded to other atom.

In the compound having a salt of a sulfonic acid group, the sulfonic acid group is preferably directly or indirectly bonded to an aromatic ring, and more preferably, is directly bonded to an aromatic ring. Due to the affinity between the organic acid and the aromatic ring of the compound having a salt of a sulfonic acid group, the organic acid and the compound having a salt of a sulfonic acid group become in close proximity to each other, and a synergistic effect between the organic acid and the salt of the sulfonic acid group of the compound having a salt of a sulfonic acid group can be enhanced, which may lead to an enhancement in the viral infection inhibitory effect against both enveloped viruses and non-enveloped viruses.

In the compound having a salt of a sulfonic acid group, when the salt of the sulfonic acid group is indirectly bonded to an aromatic ring, it is preferable that the salt of the sulfonic acid group be bonded to the aromatic ring via an alkylene group having 1 to 4 carbon atoms (preferably a methylene group or an ethylene group). While the affinity between the organic acid and the aromatic ring in the compound having a salt of a sulfonic acid group is maintained, the salt of the sulfonic acid group is moderately separated from the aromatic ring because of the alkylene group, so that the salt of the sulfonic acid group can be oriented in a more exposed state, and the viral infection inhibitor exerts an excellent viral infection inhibitory effect.

The compound having a salt of a sulfonic acid group is not particularly limited so long as the compound has one or more salts of a sulfonic acid group within a molecule. Examples thereof include a linear alkyl benzene sulfonic acid salt, an α-olefinsulfonic acid salt, an alkyl diphenyl ether sulfonic acid salt, a polyoxyalkylene alkyl ether sulfuric acid ester salt, and a polymer having a salt of a sulfonic acid group on a side chain of a linear polymer.

Examples of the linear alkylbenzene sulfonic acid salt include sodium dodecylbenzene sulfonate, calcium dodecylbenzene sulfonate, ammonium dodecylbenzene sulfonate, magnesium dodecylbenzene sulfonate, barium dodecylbenzene sulfonate, sodium tridecylbenzene sulfonate, ammonium tridecylbenzene sulfonate, sodium tetradecylbenzene sulfonate, and ammonium tetradecylbenzene sulfonate. Sodium dodecylbenzene sulfonate is preferable.

The number of carbon atoms of the alkyl group of the linear alkylbenzene sulfonic acid salt is preferably 10 or more, more preferably 11 or more, and more preferably 12 or more. The number of carbon atoms of the alkyl group of the linear alkylbenzene sulfonic acid is preferably 25 or less, more preferably 20 or less, and more preferably 18 or less. The alkyl group having the number of carbon atoms within the above-mentioned range can cause the organic acid and the compound having a salt of a sulfonic acid group to become in close proximity to each other due to the affinity between the organic acid and the hydrophobic moiety derived from the alkyl group, and a synergistic effect between the organic acid and the salt of the sulfonic acid group of the compound having a salt of a sulfonic acid group can be enhanced, so that the viral infection inhibitory effect against both the enveloped viruses and the non-enveloped viruses can be enhanced.

Examples of the α-olefinsulfonic acid salt include sodium olefinsulfonate of C12 to C18, calcium olefinsulfonate of C12 to C18, ammonium olefinsulfonate of C12 to C18, magnesium olefinsulfonate of C12 to C18, and barium olefinsulfonate of C12 to C18, and sodium C14 tetradecene sulfonate is preferable.

The number of carbon atoms of the α-olefin of the α-olefinsulfonic acid salt is preferably 12 or more, and preferably 14 or more. The number of carbon atoms of the α-olefin of the α-olefinsulfonic acid salt is preferably 22 or less, and more preferably 18 or less. The α-olefin having the number of carbon atoms within the above-mentioned range can cause the organic acid and the compound having a salt of a sulfonic acid group to become in close proximity to each other due to the affinity between the organic acid and the hydrophobic moiety derived from the α-olefin chain of the compound having a salt of a sulfonic acid group, and a synergistic effect between the organic acid and the salt of the sulfonic acid group of the compound having a salt of a sulfonic acid group can be enhanced, so that the viral infection inhibitory effect against both the enveloped viruses and the non-enveloped viruses can be enhanced.

Examples of the alkyl diphenyl ether sulfonic acid salt include a sodium salt, a calcium salt, an ammonium salt, a magnesium salt, and a barium salt of an alkyl phenyl ether having an alkyl group of C6 to C18, and sodium dodecyl diphenyl ether sulfonate of C12 is preferable.

The number of carbon atoms of the alkyl group of the alkyl diphenyl ether sulfonic acid salt is preferably 8 or more, and more preferably 10 or more. The number of carbon atoms of the alkyl group of the alkyl diphenyl ether sulfonic acid salt is preferably 24 or less, and more preferably 18 or less. The alkyl group having the number of carbon atoms within the above-mentioned range can cause the organic acid and the compound having a salt of a sulfonic acid group to become in close proximity to each other due to the affinity between the organic acid and the hydrophobic moiety derived from the alkyl group of the compound having a salt of a sulfonic acid group, and a synergistic effect between the organic acid and the salt of the sulfonic acid group of the compound having a salt of a sulfonic acid group can be enhanced, so that the viral infection inhibitory effect against both the enveloped viruses and the non-enveloped viruses can be enhanced.

In the polymer having a salt of a sulfonic acid group on a side chain of a linear polymer, the linear polymer is not particularly limited, and preferable examples thereof include a vinyl polymer, a polyester, and a polyurethane, and a vinyl polymer is preferable.

The polymer having a salt of a sulfonic acid group on a side chain of a linear polymer is not particularly limited, and examples thereof include a polymer containing a styrene sulfonic acid component, a styrene sulfonic acid salt homopolymer, a styrene-styrene sulfonic acid salt copolymer, a sulfonic acid salt of a compound obtained by sulfonating a benzene ring of a polystyrene, and a sulfonic acid salt of a compound obtained by sulfonating a benzene ring of a polymer containing a styrene component.

In addition, the polymer having a salt of a sulfonic acid group on a side chain of a linear polymer is preferably a homopolymer or a copolymer of a monomer having a salt of a sulfonic acid group. Examples of the monomer which has a salt of a sulfonic acid group include sodium p-styrene sulfonate, sodium m-styrene sulfonate, sodium o-styrene sulfonate, calcium p-styrene sulfonate, calcium m-styrene sulfonate, calcium o-styrene sulfonate, ammonium p-styrene sulfonate, ammonium m-styrene sulfonate, ammonium o-styrene sulfonate, sodium naphthalene sulfonate, and calcium naphthalene sulfonate. Sodium styrene sulfonate is preferable, and sodium p-styrene sulfonate is more preferable because of its low steric hindrance in reactivity with viruses.

In addition, the monomer having a salt of a sulfonic acid group may constitute a copolymer with other monomers. Examples of the copolymerizable monomer include an alkyl acrylate, an alkyl methacrylate, a vinyl alkyl ether, vinyl acetate, ethylene, propylene, butylene, butadiene, diisobutylene, vinyl chloride, vinylidene chloride, 2-vinylnaphthalene, styrene, acrylonitrile, acrylic acid, sodium acrylate, methacrylic acid, maleic acid, fumaric acid, maleic anhydride, acrylamide, methacrylamide, diacetone acrylamide, vinyltoluene, xylene sulfonic acid, vinylpyridine, vinylsulfonic acid, vinyl alcohol, methyl methacrylate, sodium methacrylate, and hydroxyethyl methacrylate, and styrene is preferable.

The polymer having a salt of a sulfonic acid group on a side chain of a linear polymer can be produced by a general method. Examples thereof include a method of radically polymerizing a monomer having a salt of a sulfonic acid group, a method of radically polymerizing a monomer having a salt of a sulfonic acid group and a monomer copolymerizable with the monomer, and a method of neutralizing a sulfonic acid of a polymer containing a monomer component having a salt of a sulfonic acid group using an alkali (e.g., sodium hydroxide, calcium hydroxide, potassium hydroxide, ammonium hydroxide, and the like).

### [Organic acid]

The viral infection inhibitor contains an organic acid. The viral infection inhibitor containing the organic acid promotes the release of a salt of a sulfonic acid group in the compound having such a salt of a sulfonic acid group, and enhances the viral infection inhibitory effect against non-enveloped viruses. As a result, the viral infection inhibitory effect of the viral infection inhibitor against enveloped viruses and non-enveloped viruses can be enhanced.

Furthermore, the compound having a salt of a sulfonic acid group can weaken the capsid (the shell of a protein) of non-enveloped viruses and enhance the viral infection inhibitory effect against non-enveloped viruses by the organic acid.

As described, the viral infection inhibitor contains the compound having a salt of a sulfonic acid group and the organic acid, which may lead to an enhancement in the viral infection inhibitory effect against enveloped viruses and non-enveloped viruses.

The organic acid may be any organic compound capable of promoting the release of part or all of the salts of the sulfonic acid group in the compound having a salt of a sulfonic acid group. The organic acid may be a polymer. The organic acid has a carboxy group (-COOH), a sulfonic acid group (-SO₃H), a thiol group (-SH), or a hydroxy group (-OH) within the molecule, and may have one type of these functional groups alone or a plurality of types of functional groups. It is preferable that the organic acid have a carboxy group because such an organic acid can more effectively maintain or promote the release of part or all of the salts of the sulfonic acid group in the compound having a salt of a sulfonic acid group, and can enhance the viral infection inhibitory effect against enveloped viruses and non-enveloped viruses.

The organic acid preferably has a plurality of functional groups selected from the group consisting of a carboxy group (-COOH), a sulfonic acid group (-SO₃H), a thiol group (-SH), and a hydroxy group (-OH). The organic acid more preferably has a plurality of carboxy groups. The organic acid having a plurality of functional groups can more reliably maintain or promote the release of part or all of the salts of the sulfonic acid group in the compound having a salt of a sulfonic acid group, and can further enhance the viral infection inhibitory effect against enveloped viruses and non-enveloped viruses. It is preferable that the organic acid does not contain a salt of a sulfonic acid group.

The organic acid is not particularly limited so long as it has a carboxy group (-COOH), a sulfonic acid group (-SO₃H), a thiol group (-SH), or a hydroxy group (-OH) within the molecule. Examples thereof include adipic acid (solubility: 14 g/L), benzoic acid (solubility: 3.4 g/L), lauric acid (solubility: 0 g/L), azelaic acid (solubility: 2.4 g/L), sebacic acid (solubility: 0.25 g/L), dodecanedioic acid (solubility: 0 g/L), fumaric acid (solubility: 6.3 g/L), phthalic acid (solubility: 7.2 g/L), isophthalic acid (solubility: 0.13 g/L), terephthalic acid (solubility: 0.017 g/L), methylene disalicylic acid (solubility: 0 g/L), cis-Δ4-tetrahydrophthalic acid (solubility: 0 g/L), caproic acid (solubility: 11 g/L), enanthic acid (solubility: 2.4 g/L), caprylic acid (solubility: 0.68 g/L), pelargonic acid (solubility: 0.28 g/L), capric acid (solubility: 0.15 g/L), lauric acid (solubility: 0.0048 g/L), myristic acid (solubility: 0 g/L), palmitic acid (solubility: 0 g/L), stearic acid (solubility: 0 g/L), myristoleic acid (solubility: 0 g/L), oleic acid (solubility : 0 g/L), ricinoleic acid (solubility: 0 g/L), salicylic acid (solubility: 2.0 g/L), gallic acid hydrate (solubility: 11 g/L), benzilic acid (solubility: 1.4 g/L), 4-aminobenzoic acid (solubility: 6 g/L), triglycollamic acid (solubility: 1.3 g/L), and polyacrylic acid (solubility: 250 g/L or more). Adipic acid, fumaric acid, phthalic acid, and benzoic acid are preferable, and adipic acid and benzoic acid are more preferable. It should be noted that the organic acid may be used alone or in a combination of two or more. It should be noted that the solubility described in parentheses is a solubility of an organic acid in water at 25°C.

The solubility of an organic acid in water at 25°C is preferably 20 g/L or less, and more preferably 18 g/L or less. The organic acid having a solubility in water of 20 g/L or less at 25°C enhances the affinity between the organic acid and the compound having a salt of a sulfonic acid group because of an enhancement in the hydrophobicity of the compound having a salt of a sulfonic acid group, so that the organic acid and the compound having a salt of a sulfonic acid group become in close proximity to each other. A synergistic effect between the organic acid and the salt of the sulfonic acid group of the compound having a salt of a sulfonic acid group can also be enhanced, which may lead to an enhancement in the viral infection inhibitory effect against both enveloped viruses and non-enveloped viruses. It should be noted that the solubility of an organic acid in water at 25°C refers to a mass of the organic acid dissolved in 1 L of water.

The solubility of an organic acid in water at 25°C is preferably 0.1 g/L or more, and more preferably 1 g/L or more. The organic acid having a solubility in water of 0.1 g/L or more at 25°C promotes the release of a salt of a sulfonic acid group in the compound having such a salt of a sulfonic acid group when an aqueous solution of a protein in which viruses are present, such as saliva or sputum is in contact with the viral infection inhibitor, so that the viral infection inhibitory effect against non-enveloped viruses is enhanced.

The pKa of the organic acid at 25°C is preferably 5.5 or less, more preferably 4.6 or less, and more preferably 3.8 or less. The organic acid having a pKa at 25°C of 5.5 or less enhances the viral infection inhibitory effect against enveloped viruses due to the synergistic effect between the organic acid and the salt of the sulfonic acid group. An organic acid having a pKa at 25°C of 3.8 or less further promotes the release of the salt of the sulfonic acid group to cause protein denaturation by protons, so that the viral infection inhibitory effect against not only enveloped viruses but also non-enveloped viruses is enhanced. It should be noted that the pKa of the organic acid refers to a value measured by titration at 25°C. Specifically, the pKa can be determined by performing titration at 25°C using an organic acid and sodium hydroxide, and measuring a pH value at 25°C at a half equivalence point (a point at which a half of the amount, with which neutralization is completed, is dropped).

When the organic acid is a polymer, the weight-average molecular weight of the organic acid is preferably 3000 or more, preferably 5000 or more, more preferably 10000 or more, and more preferably 100000 or more. The organic acid having a weight-average molecular weight of 3000 or more can reduce whitening and yellowing of the viral infection inhibitor. When the viral infection inhibitor is caused to adhere to the surface of a base material, such an organic acid can cause the viral infection inhibitor to more effectively exhibit the viral infection inhibitory effect without impairing the appearance of the base material. Furthermore, the organic acid having a weight-average molecular weight of 3000 or more can increase the number of virus adsorption sites per molecule of the organic acid, so that the interaction between the organic acid and the viruses becomes strong, and the viral infection inhibitory effect of the viral infection inhibitor can be enhanced.

The weight-average molecular weight of the organic acid is preferably 1000000 or less, more preferably 900000 or less, more preferably 800000 or less, and more preferably 500000 or less. The organic acid having a weight-average molecular weight of 1000000 or less can reduce yellowing of the viral infection inhibitor. When the viral infection inhibitor is caused to adhere to the surface of a base material, such an organic acid can cause the viral infection inhibitor to more effectively exhibit the viral infection inhibitory effect without impairing the appearance of the base material. Furthermore, the organic acid having a weight-average molecular weight of 1000000 or less can reduce an aggregation property of the organic acid, which may lead to a form in which the organic acid can easily interact with viruses and to an enhancement in the viral infection inhibitory effect of the viral infection inhibitor.

It should be noted that in the present invention, the weight-average molecular weight of the polymer is a value calculated as polystyrene according to GPC (gel permeation chromatography).

For example, the weight-average molecular weight can be measured by the following measuring device under measuring conditions.
Gel permeation chromatography: manufactured by Waters, trade name "2690 Separations Model"
Column: manufactured by Showa Denko K.K., trade name "GPC KF-806L"
Detector: Differential refractometer
Sample flow rate: 1 mL/min
Column temperature: 40°C
Eluent: THF

The organic acids may be particulate. The D90 particle diameter of the particulate organic acid is preferably 2 µm or more, more preferably 2.5 µm or more, more preferably 3 µm or more, and more preferably 3.5 µm or more. The D90 particle diameter of the organic acid is preferably 25 µm or less, more preferably 22 µm or less, more preferably 20 µm or less, more preferably 18 µm or less, more preferably 16 µm or less, more preferably 14 µm or less, and more preferably 12 µm or less. The organic acid with the D90 particle diameter of 2 µm or more reduces the surface area as a whole, and also reduces the aggregation property of the viral infection inhibitor, so that the organic acid and the viruses tend to easily interact with each other, and the viral infection inhibitory effect of the viral infection inhibitor is enhanced. The organic acid with the D90 particle diameter of 25 µm or less prevents aggregation of the viral infection inhibitor and increases the surface area thereof to facilitate contact with viruses, so that the viral infection inhibitory effect of the viral infection inhibitor can be enhanced. Furthermore, an organic acid having a D90 particle diameter of 25 µm or less can substantially prevent whitening caused by crystallization of the organic acid due to the interaction of the functional groups [carboxy group (-COOH), sulfonic acid group (-SO₃H), thiol group (-SH) and/or hydroxy group (-OH)] of the organic acid.

As will be described later, the D90 particle diameter of the particulate organic acid is a particle diameter (90% cumulative particle diameter) at which the cumulative frequency (accumulation from particles with a small particle diameter) in the volume-based particle size distribution by the laser scattering method is 90%. Adjustment of the D90 particle diameter of the organic acid preferably to 2 to 25 µm adjusts the particle diameter of the large particles with larger particle diameters in the organic acid to a specific range, so that coarse particles contained in the organic acid are reduced. The organic acid has a functional group [a carboxy group (-COOH), a sulfonic acid group (-SO₃H), a thiol group (-SH) and/or a hydroxy group (-OH)] within the molecule. Adjustment of the particle diameter of the organic acid to be within the above-mentioned range also adjusts the amount of the functional group present in the organic acid to impart an excellent viral infection inhibitory effect to the viral infection inhibitor, and reduces the whitening caused by the crystallization of the organic acid due to the interaction of the functional groups of the organic acid.

The D50 particle diameter of the particulate organic acid is preferably 0.5 µm or more, more preferably 1 µm or more, more preferably 1.5 µm or more, and more preferably 2.0 µm or more. The D50 particle diameter of the particulate organic acid is preferably 14 µm or less, more preferably 12 µm or less, and more preferably 11 µm or less.

In the particulate organic acid, adjustment of the D50 particle diameter within the above-mentioned range (preferably 0.5 to 14 µm) and also adjustment of the D90 particle diameter within 2 to 25 µm can reduce the coarse particles, which have the particle diameter largely away from the D50 particle diameter, contained in the organic acid, and can make the particle diameter of the organic acid be a more appropriate diameter.

Furthermore, adjustment of the particle diameter of the organic acid within a more appropriate range more appropriately adjusts the amount of the functional groups present on the surface of the organic acid, and imparts an excellent viral infection inhibitory effect to the viral infection inhibitor as well as reducing the whitening caused by the crystallization of the organic acid due to the interaction of the functional groups of the organic acid.

The D90 particle diameter and D50 particle diameter of the organic acid refer to particle diameters (90% cumulative particle diameter and 50% cumulative particle diameter) at which the cumulative frequency (accumulation from particles with a small particle diameter) in the volume-based particle size distribution by the laser scattering method are 90% and 50%, respectively. When the organic acid contains a plurality of types of organic acids, the D90 particle diameter and D50 particle diameter of the organic acid are measured based on the whole organic acid.

It is preferable that the organic acid be solid at 1 atm (1013.25 hPa) and 25°C. The organic acid, which is solid at 1 atm and 25°C, tends to appear on the surface of a viral infection-inhibiting product such as a processed coating film, and is easily brought into contact with viruses, which may lead to an enhancement in the viral infection inhibitory effect.

In the viral infection inhibitor, the content of the organic acid is preferably 5 parts by mass or more, more preferably 10 parts by mass or more, and more preferably 20 parts by mass or more, relative to 100 parts by mass of the compound having a salt of a sulfonic acid group. The organic acid contained in an amount of 5 parts by mass or more can more reliably maintain or promote the release of part or all of a salt of a sulfonic acid group in the compound having such a salt of a sulfonic acid group and can further enhance the viral infection inhibitory effect against enveloped viruses and non-enveloped viruses.

In the viral infection inhibitor, the content of the organic acid is preferably 8000 parts by mass or less, more preferably 6000 parts by mass or less, and more preferably 4000 parts by mass or less, relative to 100 parts by mass of the compound having a salt of a sulfonic acid group. The organic acid contained in an amount of 8000 parts by mass or less can enhance the viral infection inhibitory effect against both enveloped viruses and non-enveloped viruses more by the synergistic effect between the organic acid and the compound having a salt of a sulfonic acid group.

### [Viral infection inhibitor]

The viral infection inhibitor includes the compound having a salt of a sulfonic acid group and the organic acid (excluding a compound having a sulfonic acid group) as active ingredients. A method for producing the viral infection inhibitor is not particularly limited. The viral infection inhibitor can be produced by uniformly mixing, in a general manner, the compound having a salt of a sulfonic acid group and the organic acid (excluding a compound having a sulfonic acid group).

Note that the viral infection inhibitory effect refers to an effect of eliminating or reducing the ability of a virus to infect a cell, or preventing a virus from multiplying in a cell infected by the virus. Examples of a method for examining such virus infectivity include ISO 18184 and JIS L1922 for textile products, and ISO 21702 for plastics and non-porous surface products other than textile products. The Society of International sustaining growth for Antimicrobial Articles (SIAA) certifies the antiviral processing mark for products that meet the standards for the safety of antiviral processing agents and certain antiviral effects. The standard for the antiviral effects in the ISO 21702 evaluation is that a difference between a common logarithmic value of the virus infectivity titer of a blank product (product with no antiviral processing agent) and a common logarithmic value of the virus infectivity titer of a processed product (product with an antiviral processing agent) (antiviral activity value) is 2.0 or more. The viral infection inhibitor is used as a component of the antiviral processing agent. The viral infection inhibitor is, when used, kneaded into a resin or added to a surface coating agent such as a coating material, and is evaluated by the above-mentioned evaluation method.

In the present invention, for example, a product is evaluated for the viral infection inhibitory effect under the following conditions. If the difference in common logarithmic values of the virus infectivity titer (antiviral activity value) between a blank product and a processed product is 2.0 or more, the product is defined as a viral infection inhibitor. In this evaluation, regardless of the types of viruses being used for evaluation, a product having a difference in common logarithmic values of the virus infectivity titer (antiviral activity value) between a blank product and a processed product of 2.0 or more for at least one type of virus is regarded as the viral infection inhibitor.

A coating material is produced by supplying 50 mg of the viral infection inhibitor in 950 mg of a solvent-free UV-curable acrylic-based resin (manufactured by Hitachi Chemical Company, Ltd., trade name "Teslak 2328") and uniformly mixing them. After the obtained coating material is applied onto a polyester film, the coating material is irradiated with ultraviolet rays at an irradiation dose of 512 mJ/cm² using a UV conveyor device EYE GRANDAGE (manufactured by Eye Graphics Co., Ltd., irradiator reflector: cold mirror condensing type, UV lamp: H03-L31 (light emitting length 250 mm)) to cure the UV-curable acrylic-based resin, to form a coating film with a film thickness of 15 µm, which is used as a test coating film.

The obtained test coating film is subjected to an antiviral test according to ISO 21702. The virus suspension after the reaction was used to calculate the virus infectivity titer of the test coating film by the plaque method. A blank coating film is produced in the same manner as described above except that the viral infection inhibitor is not included. The virus infectivity titer (common logarithmic value) (PFU/cm²) of this blank coating film is calculated in the same manner as described above. The difference in common logarithmic values of the virus infectivity titer (antiviral activity value) is calculated by subtracting the virus infectivity titer of the test coating film from the virus infectivity titer of the blank coating film.

Other methods include a plaque method and a hemagglutination unit (HAU) measurement method described in "Medical and Pharmaceutical Virology" (first edition published in April 1990).

The viral infection inhibitor has the viral infection inhibitory effect against various types of viruses due to the synergistic effect between the organic acid and the compound having a salt of a sulfonic acid group (excluding a compound having a sulfonic acid group), and exhibits an excellent viral infection inhibitory effect against both enveloped viruses and non-enveloped viruses.

Examples of the enveloped virus include influenza virus (e.g., type A, type B, etc.), rubella virus, Ebola virus, coronavirus (e.g., SARS virus, novel coronavirus (SARS-CoV-2)), measles virus, varicella-zoster virus, herpes simplex virus, mumps virus, arbovirus, RS virus, hepatitis virus (e.g., hepatitis B virus, hepatitis C virus, etc.), yellow fever virus, AIDS virus, rabies virus, hantavirus, dengue virus, Nipah virus, and lyssa virus.

Examples of the non-enveloped virus include adenovirus, norovirus, rotavirus, human papillomavirus, poliovirus, enterovirus, coxsackievirus, human parvovirus, encephalomyocarditis virus, poliovirus, and rhinovirus.

The viral infection inhibitor is preferably used by allowing the compound having a salt of a sulfonic acid group or the organic acid, constituting the viral infection inhibitor, to be present on the surface of the resin particles. The viral infection inhibitor is preferably used by allowing the compound having a salt of a sulfonic acid group or the organic acid, constituting the viral infection inhibitor, to adhere to (be supported on) the surface of the resin particles. Furthermore, the viral infection inhibitor is more preferably used by allowing the compound having a salt of a sulfonic acid group or the organic acid, constituting the viral infection inhibitor, to adhere to (be supported on) the surface of particles. When the compound having a salt of a sulfonic acid group or the organic acid, constituting the viral infection inhibitor, is caused to adhere at least to the surface of the particles, the viral infection inhibitor can be uniformly dispersed on the base material, to be described below, without forming lumps. As a result, the surface area of the viral infection inhibitor can be increased, thereby ensuring sufficient contact between the viral infection inhibitor and the virus, which allows the viral infection inhibitor to sufficiently exhibit the viral infection inhibitory effect.

There is no particular limitation on the particles that the viral infection inhibitor, the compound having a salt of a sulfonic acid group, or the organic acid adheres to the surface thereof, as long as the viral infection inhibitory effect of the viral infection inhibitor is not inhibited. As the particles that the viral infection inhibitor, the compound having a salt of a sulfonic acid group, or the organic acid adheres to the surface thereof, resin particles and inorganic particles are preferable. Examples of synthetic resins constituting the resin particles include a styrene-based resin, an acrylic-based resin, a urethane-based resin, a vinyl chloride-based resin, an ABS resin, and synthetic rubber such as styrene-butadiene rubber (SBR) or nitrile-butadiene rubber (NBR). It is preferable to include an acrylic-based resin or a styrene-based resin, more preferably a styrene-based resin, more preferably polystyrene. It should be noted that the synthetic resin may be used alone or in a combination of two or more.

The content of the acrylic-based resin in the resin particles is preferably 50% by mass or more, more preferably 70% by mass or more, more preferably 80% by mass or more, more preferably 90% by mass or more, more preferably 95% by mass or more, and more preferably 100% by mass or more.

The content of the styrene-based resins in the resin particles is preferably 50% by mass or more, more preferably 70% by mass or more, more preferably 80% by mass or more, more preferably 90% by mass or more, more preferably 95% by mass or more, and more preferably 100% by mass or more.

The styrene-based resin is not particularly limited, and examples thereof include a homopolymer or a copolymer containing a styrene-based monomer as a monomer unit, such as styrene, methylstyrene, ethylstyrene, i-propylstyrene, dimethylstyrene, chlorostyrene, and bromostyrene, and a copolymer containing a styrene-based monomer and one or two or more vinyl monomers copolymerizable with the styrene-based monomer as monomer units.

Examples of the vinyl monomer copolymerizable with the styrene-based monomer include an acrylic monomer such as acrylonitrile, methacrylonitrile, acrylic acid, methacrylic acid, an acrylic acid ester (such as methyl acrylate, ethyl acrylate, and butyl acrylate), a methacrylic acid ester (such as methyl methacrylate, ethyl methacrylate, and butyl methacrylate), maleic anhydride, and acrylamide.

It is preferable that the synthetic resin constituting the resin particles contain an aromatic ring. An aromatic ring attracts a hydrophobic moiety of the compound having a salt of a sulfonic acid group adhering to the surface of the resin particle. Furthermore, such an aromatic ring has an action of orienting the salt of the sulfonic acid group having hydrophilicity outward, which may lead to more effective exhibition of the viral infection inhibitory effect of the viral infection inhibitor.

The inorganic material constituting the inorganic particles is not particularly limited, and examples thereof include silica, silica gel, zeolite, hydrotalcite, calcium carbonate, calcium citrate, magnesium carbonate, magnesium hydroxide, diatomaceous earth, kaolin, talc, aluminum hydroxide, titanium oxide, calcium phosphate, calcium sulfate, magnesium carbonate, zinc oxide, manganese oxide, iron oxide, aluminum oxide, barium sulfate, zirconium oxide, tungsten oxide, zirconium phosphate, zirconium carbide, glass, calcium silicate, aluminum silicate, silicon carbide, activated carbon, tobermorite, montmorillonite, bentonite, iron, tin, aluminum, zinc, copper, titanium, nickel, and various alloys. It should be noted that the inorganic material may be used alone or in a combination of two or more.

The D50 particle diameter of the particles is preferably 0.1 µm or more, and more preferably 1 µm or more. The D50 particle diameter of the particles is preferably 30 µm or less, and more preferably 15 µm or less. Particles with a D50 particle diameter of 0.1 µm or more have reduced surface area, and also lower the aggregation property of the viral infection inhibitor, so that the viral infection inhibitor and viruses tend to easily interact with each other, and the viral infection inhibitory effect is enhanced. Particles with a D50 particle diameter of 30 µm or less prevent aggregation of the viral infection inhibitor and have increased surface area to facilitate contact with viruses, so that the viral infection inhibitory effect of the viral infection inhibitor is enhanced.

Each of the D50 particle diameters of particles refers to a particle diameter (50% cumulative particle diameter) at which the cumulative frequency (accumulation from particles with a small particle diameter) in the volume-based particle size distribution by the laser scattering method is 50%. When the particles include a plurality of types of particles, the D50 particle diameter of the particles is measured based on the whole particle.

The amount of the viral infection inhibitor to adhere to the resin particles is preferably 1 part by mass or more, more preferably 5 parts by mass or more, more preferably 7 parts by mass or more, and more preferably 10 parts by mass or more, relative to 100 parts by mass of the resin particles. The viral infection inhibitor in an adhering amount of 1 part by mass or more can uniformly adhere to the surface of the resin particles, so that the viral infection inhibitory effect of the viral infection inhibitor can be more effectively exhibited.

The amount of the viral infection inhibitor to adhere to the resin particles is preferably 50 parts by mass or less, more preferably 40 parts by mass or less, more preferably 30 parts by mass or less, and more preferably 20 parts by mass or less, relative to 100 parts by mass of the resin particles. A viral infection inhibitor in an adhering amount of 50 parts by mass or less can suppress the binding of the viral infection inhibitor to itself, so that the viral infection inhibitor is efficiently disposed on the surface of the resin particles to enhance the viral infection inhibitory effect.

The amount of the compound having a salt of a sulfonic acid group to adhere to the resin particles is preferably 1 part by mass or more, more preferably 5 parts by mass or more, more preferably 7 parts by mass or more, and more preferably 10 parts by mass or more, relative to 100 parts by mass of the resin particles. The compound having a salt of a sulfonic acid group in an adhering amount of 1 part by mass or more can uniformly adhere to the surface of the resin particles, so that the viral infection inhibitory effect of the viral infection inhibitor can be more effectively exhibited.

The amount of the compound having a salt of a sulfonic acid group to adhere to the resin particles is preferably 50 parts by mass or less, more preferably 40 parts by mass or less, more preferably 30 parts by mass or less, and more preferably 20 parts by mass or less, relative to 100 parts by mass of the resin particles. The compound having a salt of a sulfonic acid group in an adhering amount of 50 parts by mass or less can suppress the binding of the compound having a salt of a sulfonic acid group together, and is efficiently disposed on the surface of the resin particles, so that the viral infection inhibitory effect is enhanced.

There is no particular limitation on how the viral infection inhibitor, the compound having a salt of a sulfonic acid group, or the organic acid adhere to the surface of the resin particles. For example, the adhesive force of the viral infection inhibitor, the compound having a salt of a sulfonic acid group, or the organic acid itself may be used, or a binder resin may be used to bond the viral infection inhibitor, the compound having a salt of a sulfonic acid group, or the organic acid to the surface of the resin particles. However, the viral infection inhibitor preferably adheres to the surface of the resin particles by the adhesive force of the compound having a salt of a sulfonic acid group itself, which is included in the viral infection inhibitor, since the viral infection inhibitory effect of the viral infection inhibitor can be effectively exhibited.

The viral infection inhibitor is used by being included in a base material to which the viral infection inhibitory effect is to be imparted. The base material including the viral infection inhibitor exhibits the viral infection inhibitory effect as a viral infection-inhibiting product.

There is no particular limitation on the base material in which the viral infection inhibitor is included as long as the viral infection inhibitor can be included. Examples of the base material include a synthetic resin molding, a coating material, a wallpaper, a decorative sheet, a floor material, a textile product (a woven fabric, a non-woven fabric, a knitted fabric), an interior good and an interior material (a seat, a child seat, a foam constituting these products, etc.) for a vehicle (e.g., an automobile, an airplane, a ship, etc.), a kitchen utensil, a baby good, and a building interior material.

As the coating material, a conventionally known coating material is used. Examples of the coating material include an oil-based coating material (e.g., a mixed paint, an oil varnish, etc.), a cellulose coating material, and a synthetic resin coating material. The coating material may include an additive such as a pigment, a plasticizer, a hardener, a bulking filler, a filler, an antioxidant, a tackifier, and a surfactant within a range in which physical properties of the coating material are not impaired. It should be noted that examples of a method for incorporating the viral infection inhibitor into a coating material include a method of supplying the viral infection inhibitor and the coating material to a dispersing device and uniformly mixing them. It should be noted that examples of the dispersing device include a high-speed mill, a ball mill, and a sand mill.

There is no particular limitation on the building interior material, and examples thereof include a floor material, a wallpaper, a ceiling material, a paint, a door knob, a switch, a switch cover, and wax.

There is no particular limitation on the vehicle interior good and vehicle interior material, and examples thereof include a seat, a child seat, a seat belt, a car mat, a seat cover, a door, a ceiling material, a floor mat, a door trim, an instrument panel, a console, a glove box, a strap, and a handrail.

### [Examples]

Hereinafter, the present invention will be described in more detail with reference to Examples, but the present invention is not limited thereto.

### [Production of carboxy group-containing compounds 1 to 5]

Predetermined amounts of acrylic acid and methyl acrylate shown in Table 1 were mixed to obtain an acrylic monomer solution. To the obtained acrylic monomer solution, 1-hydroxycyclohexan-1-ylphenyl ketone (manufactured by IGM Resins B.V., trade name "Omnirad 184") was added as a radical polymerization initiator in a predetermined amount shown in Table 1 and dissolved to produce a raw material composition.

The raw material composition was applied to a polyethylene film using a wire bar coater #14 to form a coating layer having a thickness of 35 µm. A UV conveyor device (manufactured by Eye Graphics Co., Ltd., trade name "ECS301G1") was used to irradiate the coating layer at 25°C with ultraviolet rays at a wavelength of 365 nm in an integrated amount of light of 2000 mJ/cm² to perform radical polymerization. As a result, carboxy group-containing compounds (polymers that have a carboxy group on the side chain of a linear polymer) 1 to 5 were obtained as viral infection inhibiting compounds.

**[Table 1]**

| | | Carboxy group-containing compound | | | | |
|---|---|---|---|---|---|---|
| | | 1 | 2 | 3 | 4 | 5 |
| Raw material composition (parts by mass) | Acrylic acid | 98 | 98 | 98 | 98 | 98 |
| | Methyl acrylate | 2 | 2 | 2 | 2 | 2 |
| | Radical polymerization initiator | 6 | 6 | 0.5 | 0.1 | 0.5 |

### [Production of organic acid particles]

The organic acids shown in Table 2 were each coarsely pulverized using a roll press device (Seishin Enterprise Co., Ltd., trade name "Type 150") under operating conditions of a rotation speed of 25 rpm and a pushing force of 25 t and then pulverized using a jet mill device (manufactured by Nisshin Engineering Inc., trade name "SJ-500") under operating conditions of a supply rate shown in Table 2 and a compressed air pressure of 0.75 MPa to obtain organic acid particles. It should be noted that oleic acid could not be formed in particles because its melting point is not higher than room temperature (25°C).

### [Production of resin particles with carboxy group-containing compound 2 particles adhering to surface]

Five parts by mass of particles of the carboxy group-containing compound 2 and 10 parts by mass of polystyrene particles (primary particles) having a D50 particle diameter of 4 µm were supplied to 100 parts by mass of water, and the resulting mixture was powdered using a spray dryer with an atomizer rotation speed of 20000 rpm, allowing the entire amount of the carboxy group-containing compound 2 particles to adhere to (be supported on) the surface of the polystyrene particles. Then, the mixture was pulverized using a jet mill device (manufactured by Nisshin Engineering Inc., trade name "SJ-500") under operating conditions of a raw material supply rate of 1 kg/h and a compressed air pressure of 0.75 MPa to obtain resin particles with the carboxy group-containing compound 2 particles adhering to the surface. It should be noted that, in Table 2, the "resin particles in which the carboxy group-containing compound 2 particles are adhered to surface thereof" is described as "carboxy group-containing compound 2 adhering to resin particles".

### [Production of inorganic particles with carboxy group-containing compound 2 particles adhering to surface]

Five parts by mass of particles of the carboxy group-containing compound 2 and 10 parts by mass of silica particles (primary particles) having a D50 particle diameter of 6 µm were supplied to 100 parts by mass of water, and the resulting mixture was powdered using a spray dryer with an atomizer rotation speed of 20000 rpm, allowing the entire amount of the carboxy group-containing compound 2 particles to adhere to (be supported on) the surface of the silica particles. Then, the mixture was pulverized using a jet mill device (manufactured by Nisshin Engineering Inc., trade name "SJ-500") under operating conditions of a raw material supply rate of 1 kg/h and a compressed air pressure of 0.75 MPa to obtain silica particles with the carboxy group-containing compound 2 particles adhering to the surface. It should be noted that, in Table 2, the "silica particles in which the carboxy group-containing compound 2 particles are adhered to surface thereof" is described as "carboxy group-containing compound 2 adhering to inorganic particles".

### (Examples 1 to 11 and 16 to 20, and Comparative Example 1)

One hundred parts by mass of the salt compound shown in Table 2 and 900 parts by mass of the organic acid particles shown in Table 2 were uniformly mixed to produce a viral infection inhibitor. It should be noted that, since oleic acid has a melting point of room temperature (25°C) or lower, it was used without forming particles.

### (Examples 12 to 15)

To 800 parts by mass of water, 50 parts by mass of a salt compound shown in Table 2 and 150 parts by mass of polystyrene particles (primary particles) having a D50 particle diameter of 4 µm were supplied, and powdered using a spray dryer (atomizer rotation speed: 20000 rpm), allowing the entire amount of the salt compound to adhere to (be supported on) the surface of the polystyrene particles. One hundred parts by mass of the polystyrene particles to which the salt compound adhered (was supported on) and 900 parts by mass of the organic acid particles shown in Table 2 were uniformly mixed to produce a viral infection inhibitor.

### (Example 21)

One hundred parts by mass of the salt compound shown in Table 2 and 900 parts by mass of the carboxy group-containing compound 2 particles adhering to resin particles shown in Table 2 were uniformly mixed to produce a viral infection inhibitor.

### (Example 22)

One hundred parts by mass of the salt compound shown in Table 2 and 900 parts by mass of the carboxy group-containing compound 2 particles adhering to inorganic particles shown in Table 2 were uniformly mixed to produce a viral infection inhibitor.

### (Comparative Example 2)

One hundred parts by mass of sodium dodecylbenzene sulfonate was used as a viral infection inhibitor.

### (Comparative Example 3)

One hundred parts by mass of adipic acid particles were used as a viral infection inhibitor.

Antiviral tests were performed for the obtained viral infection inhibitors using influenza virus (enveloped virus) and feline calicivirus (non-enveloped virus), and the results were shown in Table 3.

For the organic acids included in the viral infection inhibitors, the melting point at 1 atm, the solubility in water at 25°C, the pKa at 25°C, the molecular weight (weight-average molecular weight for polymers), the D90 particle diameter, and the D50 particle diameter are shown in Table 2. It should be noted that, in Table 2, the "melting point at 1 atm" and the "solubility in water at 25°C" are simply described as "melting point" and "solubility", respectively.

### (Antiviral test)

A coating material was produced by supplying 50 mg of the viral infection inhibitor in 950 mg of a solvent-free UV-curable acrylic-based resin (manufactured by Hitachi Chemical Company, Ltd., trade name "Teslak 2328") and uniformly mixing them. After the obtained coating material was applied onto a polyester film, the coating material was irradiated with ultraviolet rays at an irradiation dose of 512 mJ/cm² using UV conveyor device EYE GRANDAGE (manufactured by Eye Graphics Co., Ltd., irradiator reflector: cold mirror condensing type, UV lamp: H03-L31 (light emitting length 250 mm)) to cure the UV-curable acrylic-based resin, to form a coating film with a film thickness of 15 µm. It should be noted that in a case where the viral infection inhibitor was caused to adhere to (be supported on) the polystyrene particles or the silica particles, the amount of the viral infection inhibitor was adjusted to 50 mg.

The surface of the obtained coating film was wiped by a non-woven fabric, which was in the shape of a flat square with a side of 10 cm (manufactured by Nippon Paper Crecia Co., Ltd., trade name "Kimwipe S-200") and was soaked in 1 mL of water. The non-woven fabric was moved reciprocally 10 times on the coating film surface to wipe and thus prepare a test coating film.

The obtained test coating film was subjected to an antiviral test according to ISO 21702. The virus suspension after the reaction was used to calculate the virus infectivity titer of the test coating film by the plaque method.

A blank coating film was produced in the same manner as described above except that the viral infection inhibitor was not included, and the virus infection titer (common logarithmic value) (PFU/cm²) of this blank coating film was calculated in the same manner as described above. The virus infection titer (common logarithmic value) of the blank coating film was 6.5 PFU/cm².

The antiviral activity value was calculated by subtracting the virus infection titer of the test coating film from the virus infection titer of the blank coating film.

In the antiviral test performed in Examples, the surface of the coating film was wiped by the nonwoven fabric soaked with water in the method for measuring the virus infectivity titer described in "Description of Embodiments". The coating film after wiping is used as the test coating film.

Wiping the surface of the coating film by the above-mentioned nonwoven fabric reduces the viral infection inhibitory effect of the coating film. That is, in Examples, the virus test is performed under more stringent conditions. The antiviral activity values obtained in Examples are lower than the antiviral activity values obtained by the measurement method described in "Description of Embodiments". Thus, if the antiviral activity values obtained in the antiviral test in Examples are 2 or more, it can be determined that the viral infection inhibitors have the viral infection inhibitory effect.

### (Haze value)

A test coating film was produced in the same manner as for the antiviral test. The haze of the obtained test coating film was evaluated according to JIS K 7361. A haze value (%) was measured using a haze meter (manufactured by Murakami Color Research Laboratory Co., Ltd., trade name "HM-150") under an environment with a room temperature at 25°C and a relative humidity of 40%.

**[Table 2]**

| | Salt compound | Organic acid | | | | | | | |
|---|---|---|---|---|---|---|---|---|---|
| | | Type | Melting point (°C) | Solubility (g/L) | pKa | Molecular weight | Particle diameter (*µ* m) | | Supply rate (kg/h) |
| | | | | | | | D50 particle diameter | D90 particle diameter | |
| Example 1 | Sodium dodecylbenzenesulfonate | Adipic acid | 152 | 14 | 4.34 | 146 | 7 | 11 | 1 |
| Example 2 | Sodium dodecylbenzenesulfonate | Phthalic acid | n.d. | 7.2 | 2.94 | 166 | 6 | 12 | 1 |
| Example 3 | Sodium dodecylbenzenesulfonate | Lauric acid | 45 | 0 | 4.92 | 200 | 10 | 21 | 1 |
| Example 4 | Sodium dodecylbenzenesulfonate | Salicylic acid | 159 | 2 | 2.97 | 138 | 7 | 12 | 1 |
| Example 5 | Potassium dodecylbenzenesulphonate | Adipic acid | 152 | 14 | 4.34 | 146 | 7 | 11 | 1 |
| Example 6 | Sodium dodecyl diphenyl ether sulfonate | Phthalic acid | n.d. | 7.2 | 2.94 | 166 | 6 | 12 | 1 |
| Example 7 | Sodium dodecyl diphenyl ether sulfonate | Fumaric acid | n.d. | 6.3 | 3.02 | 116 | 7 | 10 | 1 |
| Example 8 | sodium (C14) alpha-olefin sulfonate | Adipic acid | 152 | 14 | 4.34 | 146 | 7 | 11 | 1 |
| Example 9 | Sodium polystyrene sulfonate | Adipic acid | 152 | 14 | 4.34 | 146 | 7 | 11 | 1 |
| Example 10 | Sodium dodecylbenzenesulfonate | Oxalic acid | 190 | 95 | 1.23 | 90 | 8 | 12 | 1 |
| Example 11 | Sodium dodecylbenzenesulfonate | Oleic acid | 13 | 0 | 5.35 | 282 | n.d. | n.d. | n.d. |
| Example 12 | Sodium dodecylbenzenesulfonate | Adipic acid | 152 | 14 | 4.34 | 146 | 7 | 11 | 1 |
| Example 13 | Sodium dodecyl diphenyl ether sulfonate | Fumaric acid | n.d. | 6.3 | 3.02 | 116 | 7 | 10 | 1 |
| Example 14 | Sodium polystyrene sulfonate | Phthalic acid | n.d. | 7.2 | 2.94 | 166 | 6 | 12 | 1 |
| Example 15 | Sodium polystyrene sulfonate | Fumaric acid | n.d. | 6.3 | 3.02 | 116 | 7 | 10 | 1 |
| Example 16 | Sodium dodecylbenzenesulfonate | Carboxy group-containing compound 1 | n.d. | ≥30 | 4.2-4.6 | 5000 | 2.5 | 4 | 1 |
| Example 17 | Sodium dodecylbenzenesulfonate | Carboxy group-containing compound 2 | n.d. | ≥30 | 4.2-4.6 | 5000 | 6 | 10 | 5 |
| Example 18 | Sodium dodecylbenzenesulfonate | Carboxy group-containing compound 3 | n.d. | ≥30 | 4.2-4.6 | 250000 | 6 | 10 | 1 |
| Example 19 | Sodium dodecylbenzenesulfonate | Carboxy group-containing compound 4 | n.d. | ≥30 | 4.2-4.6 | 800000 | 10 | 18 | 1 |
| Example 20 | Sodium dodecylbenzenesulfonate | Carboxy group-containing compound 5 | n.d. | 230 | 4.2-4.6 | 250000 | 15 | 30 | 5 |
| Example 21 | Sodium dodecylbenzenesulfonate | Carboxy group-containing compound 2 adhering to resin particles | n.d. | ≥30 | 4.2-4.6 | 5000 | 4 | 10 | 1 |
| Example 22 | Sodium dodecylbenzenesulfonate | Carboxy group-containing compound 2 adhering to inorganic particles | n.d. | ≥30 | 4.2-4.6 | 5000 | 7 | 11 | 1 |
| Comparative example 1 | Sodium polyoxyethylene lauryl ether acetate | Adipic acid | 152 | 14 | 4.34 | 146 | 7 | 11 | 1 |
| Comparative example 2 | Sodium dodecylbenzenesulfonate | - | - | - | - | - | - | - | - |
| Comparative example 3 | - | Adipic acid | 152 | 14 | 4.34 | 146 | 7 | 11 | 1 |

**[Table 3]**

| | Influenza virus | | Feline calicivirus | | Haze value (%) |
|---|---|---|---|---|---|
| | Virus infectivity titer common logarithmic value (PFU/cm²) | Antiviral activity value | Virus infectivity titer common logarithmic value (PFU/cm²) | Antiviral activity value | |
| Example 1 | 2.9 | 3.6 | 3.2 | 3.3 | 21 |
| Example 2 | 1.8 | 4.7 | 2.0 | 4.5 | 20 |
| Example 3 | 3.5 | 3.0 | 3.4 | 3.1 | 24 |
| Example 4 | 2 | 4.5 | 2.2 | 4.3 | 22 |
| Example 5 | 3 | 3.5 | 3.4 | 3.1 | 21 |
| Example 6 | 1.6 | 4.9 | 1.9 | 4.6 | 20 |
| Example 7 | 1.7 | 4.8 | 2.1 | 4.4 | 20 |
| Example 8 | 2.5 | 4.0 | 3.2 | 3.3 | 21 |
| Example 9 | 2.6 | 3.9 | 2.4 | 4.1 | 21 |
| Example 10 | 3.5 | 3.0 | 4.2 | 2.3 | 23 |
| Example 11 | 4 | 2.5 | 4.4 | 2.1 | 23 |
| Example 12 | 1.8 | 4.7 | 1.8 | 4.7 | 21 |
| Example 13 | 1.5 | 5.0 | 1.6 | 4.9 | 20 |
| Example 14 | 1.4 | 5.1 | 1.5 | 5.0 | 20 |
| Example 15 | 1.6 | 4.9 | 1.7 | 4.8 | 20 |
| Example 16 | 2.4 | 4.1 | 2.4 | 4.1 | 9.5 |
| Example 17 | 2.3 | 4.2 | 2.3 | 4.2 | 10 |
| Example 18 | 2.4 | 4.1 | 2.3 | 4.2 | 9.6 |
| Example 19 | 2.5 | 4.0 | 2.6 | 3.9 | 15 |
| Example 20 | 1.5 | 5.0 | 1.8 | 4.7 | 41 |
| Example 21 | 2.3 | 4.2 | 2.3 | 4.2 | 9.9 |
| Example 22 | 2.4 | 4.1 | 2.4 | 4.1 | 10 |
| Comparative example 1 | 4.2 | 2.3 | 5.8 | 0.7 | 21 |
| Comparative example 2 | 4.5 | 2.0 | 6.2 | 0.3 | 22 |
| Comparative example 3 | 4.8 | 1.7 | 6 | 0.5 | 21 |

### [Industrial applicability]

The viral infection inhibitor of the present invention including the compound having a salt of a sulfonic acid group and the organic acid has an excellent viral infection inhibitory effect against both enveloped viruses and non-enveloped viruses, and exhibits the viral infection inhibitory effect against various types of viruses.

The viral infection inhibitor of the present invention included in base materials can impart an excellent viral infection inhibitory effect to the base materials.

### (Cross-reference to related application)

The present application claims the priority under Japanese Patent Application No. 2021-040825, filed on March 12, 2021, the disclosure of which is hereby incorporated in its entirety by reference.

## Claims

1. A viral infection inhibitor comprising a compound having a salt of a sulfonic acid group and an organic acid.

2. The viral infection inhibitor according to claim 1, wherein the organic acid has a solubility of 20 g/L or less in water at 25°C.

3. The viral infection inhibitor according to claim 1 or 2, wherein the organic acid has a carboxy group.

4. The viral infection inhibitor according to any one of claims 1 to 3, wherein the organic acid has a pKa of 5.5 or less at 25°C.

5. The viral infection inhibitor according to any one of claims 1 to 3, wherein the organic acid has a pKa of 4.6 or less at 25°C.

6. The viral infection inhibitor according to claims 1 to 5, wherein the organic acid is a polymer and has a weight-average molecular weight of 3000 or more.

7. The viral infection inhibitor according to any one of claims 1 to 6, wherein the organic acid is particulate and has a D90 particle diameter of 2 to 25 µm.

8. The viral infection inhibitor according to any one of claims 1 to 7, wherein the compound having a salt of a sulfonic acid group has an aromatic ring.

9. The viral infection inhibitor according to any one of claims 1 to 8, wherein the compound having a salt of a sulfonic acid group or the organic acid is present on a surface of a particle.

10. The viral infection inhibitor according to any one of claims 1 to 8, wherein the compound having a salt of a sulfonic acid group or the organic acid adheres to a surface of a particle.

11. The viral infection inhibitor according to claim 9 or 10, wherein the particle is a resin particle or an inorganic particle.

12. The viral infection inhibitor according to claim 11, wherein the resin particle includes an acrylic-based resin or a styrene-based resin.

13. A viral infection-inhibiting product comprising:
a base material; and
the viral infection inhibitor according to any one of claims 1 to 13, the viral infection inhibitor being included in the base material.
